# EUROPEAN PATENT SPECIFICATION

(11) **EP 2 855 034 B1**
(45) Date of publication and mention of the grant of the patent: **09.09.2020**
(21) Application number: 13732256.6
(22) Date of filing: 21.05.2013
(51) Int. Cl.: B06B 1/02

(54) **ULTRASOUND TRANSDUCER ASSEMBLY AND METHOD FOR DRIVING AN ULTRASOUND TRANSDUCER HEAD**
ULTRASCHALLWANDLERANORDNUNG UND VERFAHREN ZUM ANSTEUERN EINES ULTRASCHALLWANDLERKOPFES
ENSEMBLE CAPTEUR ULTRASONORE ET PROCÉDÉ PERMETTANT DE COMMANDER UNE TÊTE DE CAPTEUR ULTRASONORE

(30) Priority: 31.05.2012 US 201261653744 P
(43) Date of publication of application: 08.04.2015
(73) Proprietor: Koninklijke Philips N.V., 5656 AG Eindhoven (NL)
(72) Inventor: SAVORD, Bernard Joseph, 5656 AE Eindhoven (NL); NNADI, Chilezie Uma, 5656 AE Eindhoven (NL); ROBINSON, Andrew Lee, 5656 AE Eindhoven (NL)
(74) Representative: Philips Intellectual Property & Standards
(86) International application number: PCT/IB2013/054158
(87) International publication number: WO 2013/179179

(56) References cited:
- WO-A1-2006/030355
- WO-A2-2008/097487
- US-A1- 2007 160 540
- US-B2- 7 252 004

## Description

### FIELD OF THE INVENTION

The present invention relates to an ultrasound transducer assembly and to a method for driving an ultrasound transducer head.

### BACKGROUND OF THE INVENTION

In the field of ultrasound imaging of shear waves it is known to use ultrasound excitations of two different energy levels as described in US7252004. A first high energy excitation called a "push pulse" is used to excite movement in the tissue. A series of subsequent low energy excitations is used to form an image of the resulting tissue motion. Image guided ultrasound therapy also requires excitations of two different energy levels, one for therapy and the other for imaging. It is desirable to perform shear wave imaging or therapy employing transducers for three dimensional imaging as described in US6013032. These transducers for 3D imaging have transmit excitation circuitry imbedded within the transducer assembly. Ultrasound imaging systems are known to have a base station and a separate transducer head connected via a flexible cable to the base station. Electrical energy is provided from the base station through the connection cable to the transmit excitation circuitry within the transducer head to drive the ultrasound transducer.

WO 2006/030355 A1 describes an integrated circuit for implementing high-voltage ultrasound functions of an ultrasound imaging system. The integrated circuit is based on silicon-on-insulator (SOI) technology and integrates high-voltage ultrasound functions such as gate-driver, power amplifier, and transmit/receive switch. Optionally, the integrated circuit may contain a low noise amplifier and an analog multiplexer.

Different ultrasound transducer excitation are known for different applications, e.g. for imaging, diagnostic, therapy and shear wave push pulse having different power consumptions. Due to the technical limitations of the power supply and the connection cable, the power spectrum provided to the transducer head is low and, therefore, the possible combination of different ultrasound transducer excitation within the transducer head is limited to the respective power consumption.

### SUMMARY OF THE INVENTION

It is an object of the present invention to provide an improved ultrasound transducer assembly comprising an ultrasound transducer head and a corresponding driving method for driving an ultrasound transducer head having a large power spectrum to drive different ultrasound transducer excitation with different power consumption.

This object is solved by the independent claims.

According to one aspect of the present invention an ultrasound transducer assembly is provided in claim 1.

According to another aspect of the present invention, a driving method for driving an ultrasound transducer head is provided in claim 14.

According to still another aspect of the present invention an ultrasound apparatus is provided comprising a base station comprising one or more power supply units and an ultrasound transducer assembly as provided according to the present invention.

Preferred embodiments of the invention are defined in the dependent claims. It shall be understood that the claimed method has similar and/or identical preferred embodiments as the claimed device and as defined in the dependent claims.

The present invention is based on the idea to provide adequate power to an ultrasound transducer head comprising different ultrasound transducer excitations having different power consumptions such that the electrical power provided from the power supply to the transducer head has to be switched from one power level to another power level. When switching from the lower power level to the higher power level, the supply voltage drops down and the electrical current in an electrical conductor increases above a current rating of the cable connector pins. By means of the charge capacitor having a capacitance larger than 100 µF the switching between the different power levels can be achieved without a significant voltage drop and without a significant increase of the current since the capacitor is slowly charged by means of the supply voltage before the power level is increased. Hence, an increased power distribution can be provided to the transducer head and, therefore, different ultrasound transducers can be integrated in the transducer head having different power consumptions.

According to the second aspect of the present invention, the voltage drop and the increase of the current during switching the lower power level to the higher power level is reduced by providing a connection cable having a reduced resistance and a reduced inductance, preferably by the factor of 4 by means of a plurality of separate parallel connection cables. Hence, the power distribution can be increased and different ultrasound transducers having a different power consumption can be integrated in the transducer head.

In general the present invention can provide adequate power to the transducer head with sufficiently high power to excite e.g. either a push pulse or a therapeutic excitation with minimum power supply droop while maintaining compatibility with a standard imaging ultrasound system designed for lower powers.

In a preferred embodiment, the ultrasound transducer assembly further comprises a controllable switch for electrically connecting the capacitor to the electrical conductor. This provides a possibility to use the capacitor only if an increased power distribution is required.

In a further embodiment the ultrasound transducer assembly further comprises a discharging element connected in parallel to the capacitor for discharging the capacitor. By means of the discharging element the charge stored in the capacitor can be removed when the capacitor is not used or the transducer is unplugged from the base station to avoid electrical shocks.

In a further embodiment, the discharging element comprises a second controllable switch and a resistor for discharging the capacitor. This provides a simple solution for discharging the capacitor and for limiting the respective discharging current.

In a preferred embodiment, the discharging element is provided for discharging the capacitor, when the capacitor is disconnected from the electrical conductor. This is a simple solution to ensure that the capacitor is discharged when the capacitor is not in use.

In a further preferred embodiment, the ultrasound transducer assembly further comprises a resistor electrically connecting the capacitor to the connector element for limiting the charge current. This is a simple solution to limit the charge current when the capacitor is charged to avoid an increase of the charge current above the current rating.

In a further embodiment, the electrical conductor comprises a plurality of parallel connection cables for connecting the capacitor to the transducer. This is a simple solution to reduce the resistance of the electrical conductor and to reduce the inductance of the electrical conductor to further reduce the voltage drop when the power level is increased.

In a preferred embodiment, the capacitor is mounted within the housing of the transducer head. This is a simple solution to reduce the size of the electrical conductor and provides a compact configuration of the ultrasound transducer assembly.

In a further preferred embodiment, the capacitor is mounted within a handle of the transducer head. This provides a further compact design and a comfortable handling of the transducer head.

In a further preferred embodiment, the capacitance of the capacitor is at least 500 µF. This is a solution to further decrease the voltage drop and to further reduce the current increase when the power level is changed.

According to a further embodiment, the ultrasound transducer head comprises an ultrasound transducer for shear wave elastography imaging. This combines two diagnostic systems and improves the examination possibilities.

According to a further embodiment, the electrical conductor is provided for transmitting electrical power at at least two different power levels for providing electrical power to the ultrasound transducer. This provides a solution to provide electrical power to the imaging transducer at different levels and to further improve the examination possibilities.

According to a further embodiment, the ultrasound transducer assembly comprises a plurality of electrical conductors for connecting the transducer head to a corresponding plurality of electrical power supplies of a base station and for transmitting electrical power from the power supplies to the transducer head at different power levels. This provides a further solution to increase the power distribution in the transducer head and to use different transducers in parallel.

According to a preferred embodiment each of the plurality of electric conductors are connected or connectable to a capacitor having a capacitance larger than 100 µF. This provides a solution to increase the power distribution in each of the conductors separately.

As mentioned above, the present invention provides a simple solution to increase a power distribution of an ultrasound transducer assembly having an ultrasound transducer head so that different transducers can be integrated in the transducer head having different power consumptions or different excitation levels can be achieved by a combined transducer.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an ultrasound system comprising a base station and a transducer head,
Fig. 2 shows a schematic circuit diagram of a known power supply and an ultrasound transducer,
Fig. 3a shows a voltage curve of the power supply shown in Fig. 2,
Fig. 3b shows a current curve of the power supply shown in Fig. 2,
Fig. 4 shows a schematic block diagram of a power supply, a connector, and an ultrasound transducer according to the present invention,
Fig. 5a shows a voltage curve of the power supply of Fig. 4, and
Fig. 5b shows a current of the power supply of Fig. 4.

### DETAILED DESCRIPTION OF THE INVENTION

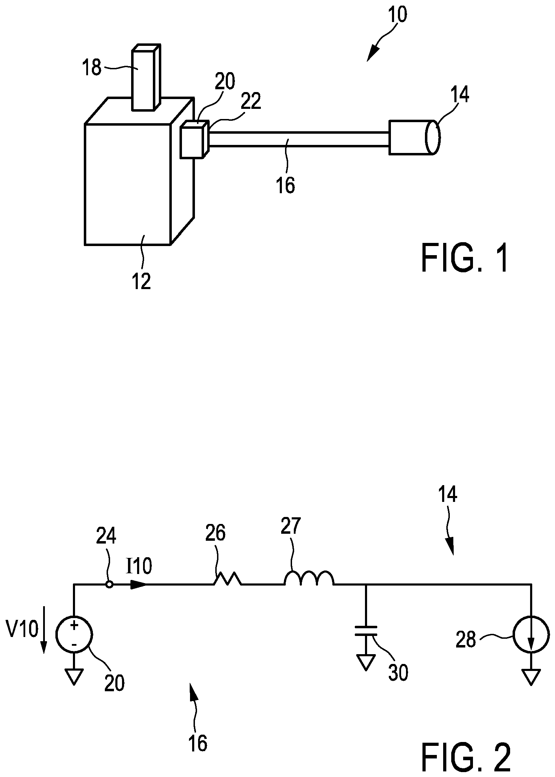

Fig. 1 shows a schematic diagram of an ultrasound system generally denoted by 10. The ultrasound system 10 comprises a base station 12 and an ultrasound transducer head 14 which are electrically connected to each other by a transducer cable 16. The base station 12 comprises a display 18. The base station comprises a power supply 20 for providing electrical power to the ultrasound transducer head 14 and a connector 22 for connecting the transducer cable 16 to the power supply 20.

The transducer head 14 comprises one or more ultrasound transducers, e.g. an ultrasound imaging transducer, an elastography ultrasound transducer, a combined transducer for shear wave elastography imaging and/or other therapy transducer elements. The power supply 20 provides electrical power via the transducer cable 16 to the ultrasound transducer in the ultrasound transducer head 14. The transducer cable 16 is a flexible cable. The base station 12 comprises the display 18 to display images e.g. provided by an ultrasound imaging transducer of the transducer head 14.

The ultrasound imaging transducer and other therapy and/or diagnostic transducer elements like an elastography ultrasound transducer are driven at different electrical power levels provided by the electrical power supply 20. The ultrasound imaging transducers usually need less than 10 W and for example an elastography ultrasound transducer needs typically 200 W to be driven during a "push" excitation. The ultrasound transducer head 14 preferably switches between driving the ultrasound imaging transducer and the elastography ultrasound transducer so that the electrical power provided from the electrical power supply 20 through the transducer cable 16 or drawn from the transducer head 14 switches from a low power level during imaging to a high power level when a push pulse is provided by the elastography transducer and from a high power level to a low power level. In case of a combined transducer for shear wave elastography imaging, the transducer is driven at two different power level for the different excitations during imaging and when a push pulse is provided.

Fig. 2 shows a schematic block diagram of the power supply 20, the transducer cable 16 and the transducer head 14. The power supply 20 provides a drive voltage V10 to a connector pin 24. The connector pin 24 connects the power supply 20 to the transducer cable 16. The transducer cable 16 comprises a resistance of approximately 2 Ohm schematically shown in Fig. 2 as a resistor 26. The transducer cable 16 comprises an inductance of approximately 4 µH generally shown in Fig. 2 as inductance 27. The transducer head 14 comprises an electrical load generally denoted by 28 which represents the ultrasound transducers. A capacitor 30 is connected in parallel to the electrical load 28 and has typically a capacitance of 4 µF.

The electrical load 28 draws a current I10 from the power supply 20 depending on the electrical power drawn by the respective ultrasound transducer. If the ultrasound system 10 is in an imaging mode, i.e. the ultrasound imaging transducer is in use, the current I10 drawn from the power supply 20 is typically 20 mA and if the elastography ultrasound transducer is in use, the current I10 drawn from the power supply 20 is typically 5 A. During the use of the ultrasound system it is frequently switched from the imaging mode to the elastography mode and from the elastography mode to the imaging mode. Each time frame in which the imaging mode is in use is typically five times longer than the elastography mode.

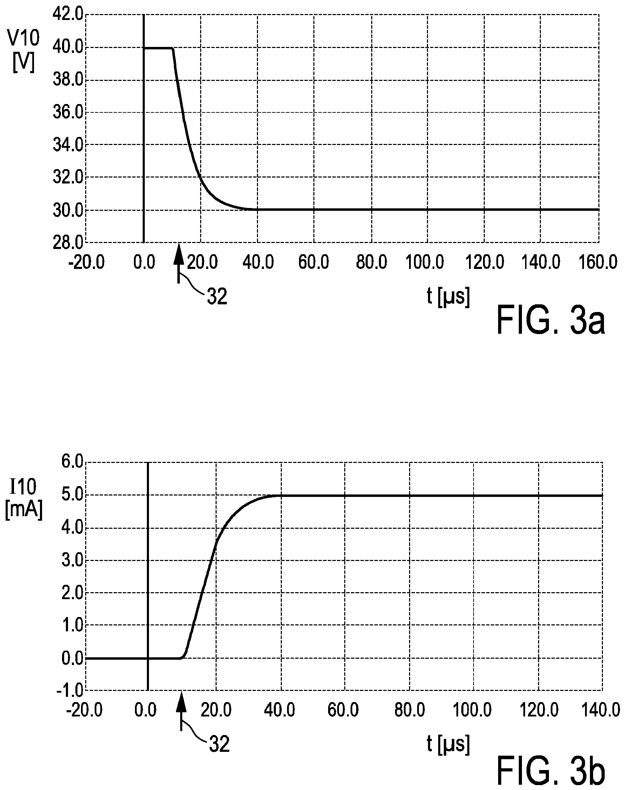

Fig. 3a and 3b show a voltage curve of the supply voltage V10 and the current I10 when the ultrasound system 10 is switched from the imaging mode to the elastography mode or in other words when the electrical power drawn from the electrical load 28 is rapidly increased from approximately 1 W to approximately 200 W. The switching time of the power levels is shown in Fig. 3a and 3b by an arrow 32. As shown in Fig. 3a, the voltage V10 drops rapidly from 40 V to 30 V when the power drawn by the electrical load 28 is increased. As shown in Fig. 3b, the current I10 increases rapidly to 5 A when the power drawn by the electrical load 28 is increased. Since the connector pin 24 typically has a current rating of 1 A and since the cable has a resistance of 2 Ohm, the power transmitted by the transducer cable 16 is limited and the electrical power drawn from the electrical load cannot be transmitted by the transducer cable 16.

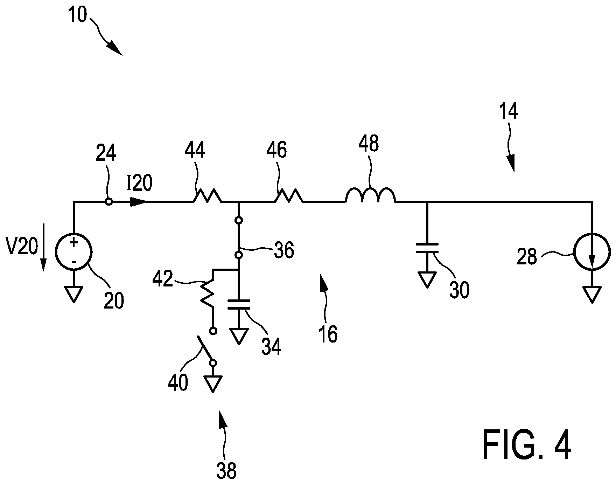

Fig. 4 shows a schematic block diagram of the ultrasound system 10 comprising the power supply 20, the transducer cable 16 and the transducer head 14. Identical elements are denoted by identical reference numerals, wherein here just the differences are explained in detail. The power supply 20 provides a drive voltage V20, which is typically 40 V and provides a drive current 120, which is dependent on the electrical load 28 and the current drawn from the electrical load 28.

A charge capacitor 34 is connected to the transducer cable 16. The charge capacitor 34 has a large capacitance of at least 100 µF, preferably 500 µF and more preferred 1000 µF. The charge capacitor 34 is connectable to the transducer cable 16 by means of a controllable switch 36. A discharge element 38 is connected in parallel to the charge capacitor 34 to discharge the capacitor 34. The discharge element 38 comprises a controllable switch 40 and a resistor 42 connected in series to each other. The charge capacitor 34 is connected to the cable 16 by means of the controllable switch 36 when the ultrasound system 10 is in use. When the ultrasound system 10 is switched off, the charge capacitor 34 is disconnected and discharged by closing the controllable switch 40. A discharge current through the resistor 42 will in this case remove the electrical charge from the charge capacitor 34 and will discharge the capacitor 34.

The transducer cable 16 comprises a resistor 44 connected between the charge capacitor 34 and the power supply 20. The resistor 44 has a resistance of typically 1.5 Ohm. The resistor 44 is a current limiting resistor to limit the charge current when the charge capacitor 34 is charged.

The transducer cable 16 is formed of a plurality of parallel separate flexible cables. Preferably the transducer cable 16 is formed by three, four, five, six or more cables to reduce the resistance of the transducer cable 16 by the factor of three, four, five, six or more and to reduce the inductance of the transducer cable 16. The resistance of the parallel cables is generally shown in Fig. 4 by a resistor 46. The resistor 46 has a resistance of typically 0.5 Ohm. The inductance of the parallel cables is generally shown in Fig. 4 by an inductor 48 which is approximately 1 µH.

When the ultrasound system 10 is switched on and the controllable switch 36 is closed, the charge capacitor 34 is charged via the current limiting resistor 44. During the imaging mode, a low current 120 is drawn from the electrical load 28. When the power drawn from the electrical load 28 is increased to a high level, the voltage drop of the drive voltage V20 is reduced due to the charged capacitor 34 as shown below. Further, the current 120 is slowly increased due to the charged capacitor 34 and due to the current limiting resistor 44 and is kept below the current rating of the input pin 24. During the use the ultrasound system 10 is in the imaging mode and frequently for a short time frame switched to the elastography mode to provide an acoustic push. Hence, the power level drawn from the transducer head 14 switches frequently from a low power level to a high power level.

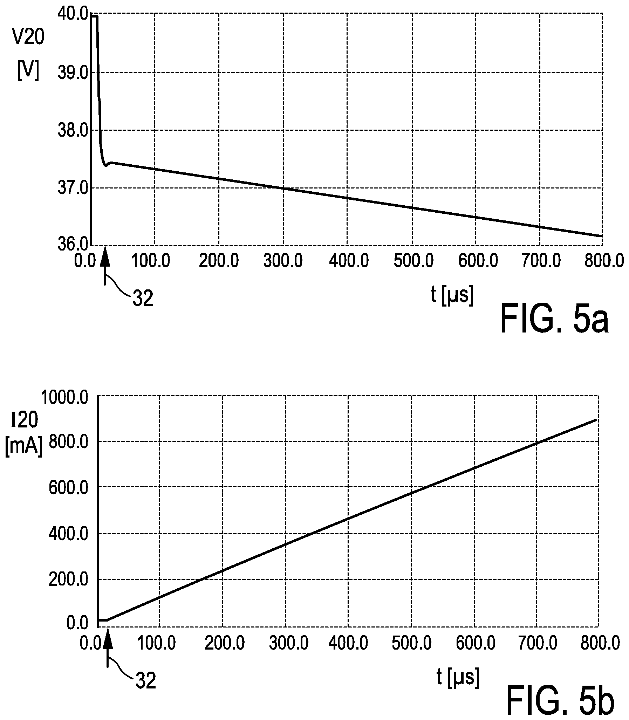

Fig. 5a shows a voltage curve of the supply voltage V20 of Fig. 4 when the power drawn by the electrical load 28 is increased. Further, Fig. 5b shows the current 120 from Fig. 4 when a power drawn by the electrical load 28 is increased. As shown in Fig. 5a the voltage V20 drops from 40 V to approximately 37 V and the voltage drop keeps below 10 %. The current 120 shown in Fig. 5b increases linearly up to approximately 1 A during the power pulse so that the current rating is not reached. Hence, by means of the charge capacitor 34, the voltage drop can be reduced, the current can be kept below the current rating and the necessary power of in this case 200 W can be transmitted from the power supply 20 to the transducer head 14 via the transducer cable 16.

The charge capacitor 34 and the discharge element 38 are preferably mounted at the end of the transducer cable 16 with the system connector 22. E.g. the charge capacitor 34 and the discharge element 38 are mounted in a housing of the connector. In an alternative embodiment, the charge capacitor 34 and the discharge element 38 are mounted in the transducer head 14.

In a further preferred embodiment, the base station 20 comprises a plurality of power supplies 20 which are each connected by a separate transducer cable 16 to the transducer head 14 and each comprises a separate charge capacitor 34. In this embodiment different power levels can be provided by means of different transducer cables 16 to the transducer head and also drive the different ultrasound transducers in the transducer head in parallel.

In a further embodiment the charge capacitor 34 has a capacitance of more than approximately 2500 µF, preferably 2700 µF.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art without departing from the scope of the invention as defined by the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Ultrasound transducer assembly (10), comprising:
- an ultrasound transducer head (14),
- a transducer cable (16) for connecting the transducer head (14) to an electrical power supply (20) of a base station (12) and for transmitting electrical power from the power supply (20) to the transducer head (14), and
- a connector element (22) for connecting the transducer cable (16) to the power supply (20) and for receiving an input voltage (V20) from the power supply (20), **characterized in that** the ultrasound transducer assembly further comprises:
- a capacitor (34) electrically connectable to the transducer cable (16) for storing electrical charge, wherein the capacitor (34) has a capacitance larger than or equal to 100 µF,
- a controllable switch (36) for electrically connecting the capacitor (34) to the transducer cable (16), and
- a discharging element (38) connected in parallel to the capacitor (34) for discharging the capacitor (34), the discharging element (38) comprising a second controllable switch (40) and a resistor (42) connected in series with each other.

2. Ultrasound transducer assembly as claimed in claim 1, wherein the discharging element (38) is adapted to discharge the capacitor (34) when the capacitor (34) is disconnected from the transducer cable (16).

3. Ultrasound transducer assembly as claimed in claim 1 or 2, further comprising a resistor (44) electrically connecting the capacitor (34) to the connector element (22) for limiting a charge current (120).

4. Ultrasound transducer assembly as claimed in any of claims 1 to 3, wherein the transducer cable (16) comprises a plurality of separate parallel connection cables (46, 48) for connecting the capacitor (34) to the transducer head (14).

5. Ultrasound transducer assembly as claimed in any of claims 1 to 4, wherein the capacitor (34) is mounted within a housing of the transducer head (14).

6. Ultrasound transducer assembly as claimed in any of claims 1 to 5, wherein the capacitor (34) is mounted within a handle of the transducer head (14).

7. Ultrasound transducer assembly as claimed in any of claims 1 to 6, wherein the capacitance of the capacitor (34) is at least 500 µF.

8. Ultrasound transducer assembly as claimed in any of claims 1 to 6, wherein the capacitance of the capacitor (34) is more than 2500 µF.

9. Ultrasound transducer assembly as claimed in any of claims 1 to 8, wherein the ultrasound transducer head (14) comprises an ultrasound transducer for shear wave elastography imaging.

10. Ultrasound transducer assembly as claimed in claim 9, wherein the electrical conductor (16) is provided for transmitting electrical power at at least two different power levels for providing electrical power to the ultrasound transducer.

11. Ultrasound transducer assembly as claimed in any of claims 1 to 10, comprising a plurality of electrical conductors (16) for connecting the transducer head (14) to a corresponding plurality of electrical power supplies (20) of the base station (12) and for transmitting electrical power from the power supplies (20) to the transducer head (14) at different power levels.

12. Ultrasound apparatus, comprising:
- one or more power supply units; and
- an ultrasound transducer assembly as claimed in any of claims 1 to 11.

13. Method for driving an ultrasound transducer head (14) comprising the steps of:
- connecting the transducer head (14) to a power supply (20) by means of an transducer cable (16);
- providing a charge capacitor (34) having a capacitance larger than or equal to 100 µF, the charge capacitor (34) being electrically connectable to the transducer cable (16);
- providing and controlling a controllable switch (36) for electrically connecting the charge capacitor (34) to the transducer cable (16);
- connecting a discharging element (38) in parallel to the charge capacitor (34) for discharging the charge capacitor (34) wherein the discharging element (38) comprises a second controllable switch (40) and a resistor (42) connected in series with each other;
- providing electrical power from the power supply (20) to the ultrasound transducer head (14) at a first power level; and
- providing electrical power from the power supply (20) to the ultrasound transducer head (14) at a second power level higher than the first power level.

## Patentansprüche

1. Ultraschallwandler-Baugruppe (10), die Folgendes umfasst:
- einen Ultraschallkopf (14),
- ein Wandlerkabel (16) zum Anschließen des Schallkopfs (14) an der Stromversorgung (20) einer Basisstation (12) sowie zum Übertragen des elektrischen Stroms von der Stromversorgung (20) zum Schallkopf (14), und
- ein Steckerelement (22) zum Anschließen des Wandlerkabels (16) an der Stromversorgung (20) sowie zum Aufnehmen einer Eingangsspannung (V20) von der Stromversorgung (20),
und die sich dadurch auszeichnet, dass die Ultraschallwandler-Baugruppe zudem Folgendes umfasst:
- einen Kondensator (34), der elektrisch am Wandlerkabel (16) angeschlossen wird, um elektrische Ladung zu speichern, wobei der Kondensator (34) über eine Kapazität verfügt, die mindestens 100 µF entspricht,
- einen steuerbaren Schalter (36) für den elektrischen Anschluss des Kondensators (34) am Wandlerkabel (16), und
- ein parallel zum Kondensator (34) geschaltetes Entdladungselement (38) zum Entladen des Kondensators (34), wobei das Entladungselement (38) über einen zweiten steuerbaren Schalter (40) sowie einen Widerstand (42) verfügt, die in Reihe zueinander geschaltet sind.

2. Ultraschallwandler-Baugruppe gemäß Anspruch 1, wobei das Entladungselement (38) den Kondensator (34) entlädt, wenn der Kondensator (34) vom Wandlerkabel getrennt wird (16).

3. Ultraschallwandler-Baugruppe gemäß Anspruch 1 oder 2,
wobei diese zudem über einen Widerstand (44) verfügt, der den Kondensator (34) elektrisch am Steckerelement (22) anschließt, um den Ladestrom (120) zu begrenzen.

4. Ultraschallwandler-Baugruppe gemäß einer der Ansprüche 1 bis 3, wobei das Wandlerkabel (16) über mehrere separate parallele Verbindungskabel (46, 48) zum Anschließen des Kondensators (34) am Schallkopf (14) verfügt.

5. Ultraschallwandler-Baugruppe gemäß einer der Ansprüche 1 bis 4, wobei der Kondensator (34) im Gehäuse des Schallkopfs (14) angebracht ist.

6. Ultraschallwandler-Baugruppe gemäß einer der Ansprüche 1 bis 5, wobei der Kondensator (34) in einem Griff des Schallkopfs (14) angebracht ist.

7. Ultraschallwandler-Baugruppe gemäß einer der Ansprüche 1 bis 6, wobei die Kapazität des Kondensators (34) mindestens 500 µF beträgt.

8. Ultraschallwandler-Baugruppe gemäß einer der Ansprüche 1 bis 6, wobei die Kapazität des Kondensators (34) mehr als 2.500 µF *beträgt.*

9. Ultraschallwandler-Baugruppe gemäß einer der Ansprüche 1 bis 8, wobei der Ultraschallkopf (14) über einen Ultraschallwandler für die Scherwellen-Elastographie-Bildgebung verfügt.

10. Ultraschallwandler-Baugruppe gemäß Anspruch 9, wobei der elektrische Leiter (16) elektrischen Strom mit mindestens zwei verschiedenen Leistungspegeln liefert, um den Ultraschallwandler mit elektrischem Strom zu versorgen.

11. Ultraschallwandler-Baugruppe gemäß einer der Ansprüche 1 bis 10, die über mehrere elektrische Leiter (16) zum Anschließen des Schallkopfs (14) an den entsprechenden Stromversorgungen (20) der Basisstation (12) sowie zum Übertragen von elektrischem Strom mit verschiedenen Leistungspegeln von den Stromversorgungen (20) zum Schallkopf (14) verfügt.

12. Ultraschallapparat, der Folgendes umfasst:
- mindestens ein Netzteil; und
- eine Ultraschallwandler-Baugruppe gemäß einer der Ansprüche 1 bis 11.

13. Methode zum Betreiben eines Ultraschallkopfs (14), die folgende Schritte umfasst:
- Anschließen des Schallkopfs (14) an der Stromversorgung (20) mit einem Wandlerkabel (16);
- Bereitstellen eines Ladekondensators (34) mit einer Kapazität von mindestens 100 µF, wobei der Ladekondensator (34) elektrisch am Wandlerkabel (16) angeschlossen werden kann;
- Bereitstellen und Steuern eines steuerbaren Schalters (36) für den elektrischen Anschluss des Ladekondensators (34) am Wandlerkabel (16);
- paralleles Schalten eines Entdladungselements (38) und des Ladekondensators (34) zum Entladen des Ladekondensators (34), wobei das Entladungselement (38) über einen zweiten steuerbaren Schalter (40) sowie einen Widerstand (42) verfügt, die in Reihe zueinander geschaltet sind;
- Bereitstellen von elektrischem Strom von der Stromversorgung (20) zum Ultraschallkopf (14) mit einem ersten Leistungspegel; und
- Bereitstellen von elektrischem Strom von der Stromversorgung (20) zum Ultraschallkopf (14) mit einem zweiten Leistungspegel, der höher ist als der erste Leistungspegel.

## Revendications

1. Ensemble transducteur à ultrasons (10), comprenant :
- une tête de transducteur à ultrasons (14),
- un câble de transducteur (16) pour raccorder la tête de transducteur (14) à une alimentation électrique (20) d'une station de base (12) et pour transmettre une puissance électrique de l'alimentation électrique (20) à la tête de transducteur (14), et
- un élément connecteur (22) pour raccorder le câble de transducteur (16) à l'alimentation électrique (20) et pour recevoir une tension d'entrée (V20) de l'alimentation électrique (20),
**caractérisé en ce que** l'ensemble transducteur à ultrasons comprend en outre :
- un condensateur (34) pouvant être raccordé électriquement au câble transducteur (16) pour stocker une charge électrique, dans lequel le condensateur (34) présente une capacité supérieure ou égale à 100 pF,
- un interrupteur (36) commandable pour raccorder électriquement le condensateur (34) au câble de transducteur (16), et
- un élément de décharge (38) raccordé en parallèle au condensateur (34) pour décharger le condensateur (34), ledit élément de décharge (38) comprenant un second interrupteur (40) commandable et une résistance (42) raccordés en série l'un à l'autre.

2. Ensemble transducteur à ultrasons selon la revendication 1, dans lequel
l'élément de décharge (38) est conçu pour décharger le condensateur (34) lorsque le condensateur (34) est débranché du câble de transducteur (16).

3. Ensemble transducteur à ultrasons selon la revendication 1 ou 2, comprenant en outre une résistance (44) raccordant électriquement le condensateur (34) à l'élément connecteur (22) pour limiter un courant de charge (120).

4. Ensemble transducteur à ultrasons selon l'une quelconque des revendications 1 à 3, dans lequel le câble de transducteur (16) comprend une pluralité de câbles de raccordement parallèles séparés (46, 48) pour raccorder le condensateur (34) à la tête de transducteur (14).

5. Ensemble transducteur à ultrasons selon l'une quelconque des revendications 1 à 4, dans lequel le condensateur (34) est monté à l'intérieur d'un boîtier de la tête de transducteur (14).

6. Ensemble transducteur à ultrasons selon l'une quelconque des revendications 1 à 5, dans lequel le condensateur (34) est monté à l'intérieur d'une poignée de la tête de transducteur (14).

7. Ensemble transducteur à ultrasons selon l'une quelconque des revendications 1 à 6, dans lequel la capacité du condensateur (34) est d'au moins 500 pF.

8. Ensemble transducteur à ultrasons selon l'une quelconque des revendications 1 à 6, dans lequel la capacité du condensateur (34) est supérieure à 2500 pF.

9. Ensemble transducteur à ultrasons selon l'une quelconque des revendications 1 à 8, dans lequel la tête de transducteur à ultrasons (14) comprend un transducteur à ultrasons pour l'imagerie par élastographie par ondes de cisaillement.

10. Ensemble transducteur à ultrasons selon la revendication 9, dans lequel le conducteur électrique (16) est fourni pour transmettre une puissance électrique à au moins deux niveaux de puissance différents pour fournir une puissance électrique au transducteur à ultrasons.

11. Ensemble transducteur à ultrasons selon l'une quelconque des revendications 1 à 10, comprenant une pluralité de conducteurs électriques (16) pour raccorder la tête de transducteur (14) à une pluralité correspondante d'alimentations électriques (20) de la station de base (12) et pour transmettre de l'énergie électrique des alimentations électriques (20) à la tête de transducteur (14) à différents niveaux de puissance.

12. Appareil à ultrasons, comprenant :
- au moins une unité d'alimentation électrique ; et
- un ensemble transducteur à ultrasons selon l'une quelconque des revendications 1 à 11.

13. Procédé d'entraînement d'une tête de transducteur à ultrasons (14) comprenant les étapes suivantes :
- le raccordement de la tête de transducteur (14) à une alimentation électrique (20) au moyen d'un câble de transducteur (16) ;
- la fourniture d'un condensateur de charge (34) présentant une capacité supérieure ou égale à 100 pF, ledit condensateur de charge (34) pouvant être raccordé électriquement au câble de transducteur (16) ;
- la fourniture et la commande d'un interrupteur (36) commandable pour raccorder électriquement le condensateur de charge (34) au câble de transducteur (16) ;
- le raccordement d'un élément de décharge (38) en parallèle au condensateur de charge (34) pour décharger le condensateur de charge (34), dans lequel l'élément de décharge (38) comprend un second interrupteur (40) commandable et une résistance (42) raccordés en série l'un à l'autre ;
- la fourniture de l'énergie électrique de l'alimentation électrique (20) à la tête de transducteur à ultrasons (14) à un premier niveau de puissance ; et
- la fourniture de l'énergie électrique de l'alimentation électrique (20) à la tête de transducteur à ultrasons (14) à un second niveau de puissance supérieur au premier niveau de puissance.
